# EUROPÄISCHE PATENTANMELDUNG

(11) **EP 3 984 371 A1**
(43) Veröffentlichungstag der Anmeldung: **20.04.2022**
(21) Anmeldenummer: 21203282.5
(22) Anmeldetag: 18.10.2021
(51) Int. Cl.: A23D 7/04, A23D 9/05, A23J 1/08, A23L 3/015, A23L 3/10, A23P 10/25, A23P 10/35, A23B 5/015, A23L 3/32, A23L 33/17, A61K 35/57, A61K 38/17

(54) **VERFAHREN ZUR VERKAPSELUNG VON NATÜRLICHEM FOLLISTATIN**

(30) Priorität: 16.10.2020 DE 102020213091
(71) Anmelder: Deutsches Institut für Lebensmitteltechnik e.V., 49610 Quakenbrück (DE)
(72) Erfinder: Volker, Lammers, 49610 Quakenbrück (DE); Volker, Heinz, 49610 Quakenbrück (DE); Pajic, Aleksandar, 49610 Quakenbrück (DE); Rumker von Höven, Matthias, 49610 Quakenbrück (DE); Bommes, Caroline, 49610 Quakenbrück (DE)
(74) Vertreter: Taruttis, Stefan Georg

(57) **Zusammenfassung**

Die Erfindung betrifft ein Verfahren zur Herstellung von mit Fett verkapseltem Follistatin, das die Schritte a) Dosieren von Fett, das einen Schmelzpunkt von zumindest 40°C aufweist, in einen Extruder, b) Erwärmen des Fetts in dem Extruder bei Drehung der zumindest einen Schnecke, c) Zudosieren des Follistatins in das Extrudergehäuse zur Herstellung einer fließfähigen Mischung, d) Kühlen der fließfähigen Mischung in einem stromabwärts angrenzenden Abschnitt des Extrudergehäuses, e) anschließendes Austreten der Mischung durch eine Extruderdüse und f) Zerkleinern der Mischung nach Austritt aus der Extruderdüse aufweist.

## Beschreibung

Die vorliegende Erfindung betrifft ein Verfahren zur Verkapselung von natürlichem Follistatin, insbesondere zur Verwendung in Nahrungsmitteln und Futtermitteln, wobei das natürliche Follistatin in trockener Form, in fester oder in flüssiger Zusammensetzung vorliegen kann. Das verkapselte Follistatin ist insbesondere bei 20°C bis 40°C oder bis 38°C fest oder flüssig, und das mit dem Verfahren erhältliche verkapselte Follistatin ist als Zusatz für Nahrungsmittel und Futtermittel verwendbar. Das Follistatin kann in Form von befruchteten Vogeleiern vorliegen, insbesondere befruchteten Hühnereiern, als Vollei, Eigelb oder Eiklar aus befruchteten Vogeleiern und/oder in Form von rohem tierischen Blutserum vorliegen, optional mit dem Schritt des Aufkonzentrierens einer Follistatin enthaltenden Fraktion aus dem Vollei, Eigelb oder Eiklar und/oder tierischem Blutserum durch Fällung einer löslichen Proteinfraktion, die Follistatin enthält, und Abtrennen der gefällten Fraktion vor dem Konservieren des Follistatins und vor dem Verkapseln. Das Konservieren, das ein Sterilisieren des Follistatin enthaltenden Volleis, Eigelbs oder Eiklars aus befruchteten Vogeleiern und/oder des Follistatin enthaltenden rohen tierischen Blutserums ist, erfolgt z.B. durch Gammabestrahlung, bevorzugt durch Hochdruckbehandlung und/oder durch Behandlung mit gepulsten elektrischen Feldern.

Das Verfahren zeichnet sich dadurch aus, dass im Verhältnis zum Verkapselungsmaterial ein sehr hoher Volumenanteil an Follistatin, in trockener oder flüssiger Form, verkapselt wird und das Verfahren mit geringem maschinellen Aufwand durchführbar ist. Das erhältliche verkapselte Follistatin zeichnet sich bevorzugt dadurch aus, dass es einen geringen Gehalt an Verkapselungsmaterial aufweist. Das verkapselte Follistatin zeichnet sich insbesondere dadurch aus, dass es bei Raumtemperatur stabil ist und ohne Zusatzstoff rieselfähig und leicht dosierbar ist. Optional ist das verkapselte Follistatin magensaftresistent, so dass das Follistatin erst nach Eintritt in den Dünndarm bzw. während der Passage des Dünndarms aus der Verkapselung freigesetzt wird.

Weiter betrifft die Erfindung die Verwendung des verkapselten Follistatins zur Verbesserung des Muskelzuwachses und/oder der Muskelregeneration, insbesondere durch Verabreichung des verkapselten Follistatins als Zusatz zur Nahrung.

Die EP2806745 B1 beschreibt zur Herstellung biologisch aktiven Follistatins die Behandlung von rohem flüssigen Eigelb, Vollei oder Eiklar aus befruchteten Vogeleiern oder von rohem tierischen Blutserum mit Hochdruck von wenigstens 4500 bar oder mit gepulsten elektrischen Feldern von wenigstens 5 kV/cm bei einer Fließrate von 30 L/h. Die EP 2 883 461 B1 beschreibt zur Herstellung biologisch aktiven Follistatins, dass aus Eigelb, Vollei oder Eiklar aus befruchteten Vogeleiern oder aus rohem tierischen Blutserum zunächst eine Follistatin enthaltende Fraktion gefällt wird, die anschließend durch Hochdruck oder gepulste elektrische Felder behandelt wird.

Brodkorb et al, Nature Protocols 2019 (https://doi.org/10.1038/s41596-018-0119-1) beschreiben einen in vitro Test zur Simulation der Verdauung.

Der Erfindung stellt sich die Aufgabe, ein alternatives Verfahren zum Herstellen einer bei Raumtemperatur lagerstabilen Formulierung von natürlichem Follistatin anzugeben, sowie alternatives lagerstabiles und konserviertes Follistatin, vorzugsweise eine Formulierung von natürlichem Follistatin, die magensaftresistent ist und biologisch verfügbares Follistatin bereitstellt. Bevorzugt soll das Verfahren einfach durchführbar sein und haltbares Follistatin herstellen, bevorzugt ohne einen Schritt des Trocknens des Follistatin enthaltenden Rohstoffs. Weiter bevorzugt soll das konservierte Follistatin bei Raumtemperatur rieselfähig sein, leicht dosierbar sein, und/oder die Formulierung soll Follistatin nach dem Verzehr erst nach Eintritt in den Dünndarm freisetzen.

Die Erfindung löst die Aufgabe mit den Merkmalen der Ansprüche und stellt insbesondere ein Verfahren zur Herstellung von mit Fett verkapseltem Follistatin bereit, das die Schritte
a) Dosieren von Fett, das optional eine Fettmischung ist, mit einem Schmelzpunkt von zumindest 40 °C, bevorzugt von zumindest 50 °C, bevorzugter zumindest 55 °C, z.B. 50 bis 90 °C, bevorzugt 55 bis 70 °C, bevorzugter zumindest 60 bis 70 °C in einen Extruder,
b) bei Drehung der zumindest einen Schnecke des Extruders, Erwärmen des Fetts, z.B. auf eine Temperatur, bei der das Fett fließfähig ist, die z.B. 35 bis 40 K oberhalb der Schmelztemperatur des Fetts liegt, z.B. maximal 30 K oder maximal 25 K oder maximal 20 K oder maximal 10 K oberhalb der Schmelztemperatur des Fetts, optional bis auf maximal die Schmelztemperatur des Fetts, zur Erzeugung fließfähigen Fetts,
c) Zudosierung des Follistatins in das Extrudergehäuse, bevorzugt stromabwärts der Fettdosierung oder an der gleichen Stelle wie die Fettdosierung, und Vermischen des Follistatins mit dem Fett im Extrudergehäuse zur Herstellung einer fließfähigen Mischung, die das Fett und Follistatins enthält oder daraus besteht,
d) Kühlen der fließfähigen Mischung im Extruder auf die Schmelztemperatur des Fetts oder darunter, so dass z.B. eine viskose, aber noch fließfähige Konsistenz erreicht wird, bevorzugt durch Kühlen der Mischung auf eine Temperatur, die in einem Bereich von zumindest 1 K, zumindest 2 K, zumindest 3 K, zumindest 4 K oder zumindest 5 K, bevorzugt zumindest 10 K, bevorzugter zumindest 15 K, bevorzugter zumindest 20 K oder zumindest 25 K oder zumindest 30 K, z.B. zumindest 35 bis 50 K oder bis 40 K unterhalb der Schmelztemperatur des Fetts oder der Mischung liegt,
e) unmittelbar anschließendes Pressen bzw. Extrudieren der Mischung durch eine Extruderdüse, die z.B. Düsenöffnungen mit einem Durchmesser von 250 µm bis 6 mm, bevorzugter 500 µm bis 3 mm oder bis 2 mm oder bis 1 mm aufweist, zur Herstellung zumindest eines Extrudatstranges und,
f) nach Austritt aus der Extruderdüse, Zerkleinern des Extrudatstranges, z.B. durch Schneiden, zur Herstellung von Pellets aus der Mischung,
g) optional anschließendes Trocknen der Pellets, z.B. bis die Oberfläche trocken ist, bevorzugt bei einer Temperatur unterhalb der Schmelztemperatur des Fetts,
h) optional und bevorzugt anschließendes Behandeln der Pellets durch Temperieren auf eine Temperatur von zumindest 2 bis 10 K unterhalb der Schmelztemperatur des Fetts und zumindest 50 °C, bevorzugt zumindest 55 °C, bevorzugter zumindest 58 °C, noch bevorzugter zumindest 60 °C, für zumindest 1 d, bevorzugter 3 bis 10 d, z.B. 5 bis 7 d, aufweist oder daraus besteht.

Generell wird im Verfahren, zumindest während der Schritte a), b), c), d) und e), die zumindest eine Schnecke des Extruders ohne Unterbrechung gedreht.

Optional wird nach dem Erwärmen des Fetts, das in Schritt a) dosiert wird und z.B. einen Schmelzpunkt von zumindest 50 °C aufweist, auf zumindest seine Schmelztemperatur in Schritt b) und vor dem Kühlen der fließfähigen Mischung im Extruder in Schritt d) ein niedrigschmelzendes Fett in den Extruder dosiert. Das niedrigschmelzende Fett wird bevorzugt zu maximal 20 Gew.-%, bevorzugter zu maximal 19 Gew.-%, zu maximal 18 Gew.-%, zu maximal 17 Gew.-%, noch bevorzugter zu maximal 16 Gew.-% der Summe der Fette zudosiert. Dabei wird das niedrigschmelzende Fett z.B. zu zumindest 5 Gew.-% oder zumindest 10 Gew.-% oder zumindest 12 Gew.-% der Summe der Fette zudosiert. Die Summe der Fette ist die Masse des in Schritt a) dosierten Fetts z.B. mit einem Schmelzpunkt von zumindest 50 °C, und des niedrigschmelzenden Fetts. Das niedrigschmelzende Fett hat einen Schmelzpunkt von maximal 40 °C, bevorzugt von maximal 39 °C, von maximal 38 °C oder von maximal 37 °C, maximal 36 °C oder maximal 35 °C. Optional kann das niedrigschmelzende Fett eines sein, das bei Raumtemperatur flüssig ist, z.B. ungehärtetes Pflanzenöl.

Das Fett, das in Schritt a) dosiert wird, wird bevorzugt als Feststoff, z.B. als Pulver oder Granulat, in den Extruder dosiert. Das niedrigschmelzende Fett wird bevorzugt flüssig in den Extruder dosiert.

Gegenwärtig wird angenommen, dass das Follistatin erst nach Passieren des Magens im Dünndarm durch Lipasen aus dem verkapselten Follistatin freigesetzt wird, weil die Schmelztemperatur des als Verkapselungsmittel verwendeten Fetts über der Körpertemperatur von Menschen, Nutztieren und Haustieren liegt. Dies ist für Follistatin vorteilhaft, da es im Magen durch Säure und Verdauungsenzyme angegriffen werden kann und erst nach Passage des Magens, z.B. im Dünndarm freigesetzt werden soll. Bevorzugt findet das verkapselte Follistatin Verwendung als Nahrungsmittel oder Arzneimittel für Menschen oder monogastrische Tiere, insbesondere monogastrische Nutztiere oder Haustiere, z.B. zur Behandlung von oder Vorbeugung gegen Muskelschwund.

Bei Dosierung eines niedrigschmelzenden Fetts in den Extruder konnten Pellets hergestellt werden, die das niedrigschmelzende Fett enthalten und dennoch erst nach einer Zeit und Temperatur, die der Passage in den Dünndarm entspricht, den Inhaltsstoff freisetzen. Es wird angenommen, dass das niedrigschmelzende Fett das Auflösen des Fetts, das in Schritt a) dosiert wird und z.B. einen Schmelzpunkt von zumindest 50 °C hat, bechleunigt.

Es hat sich gezeigt, dass bei der Zudosierung eines niedrigschmelzenden Fetts nach Schritt b) und vor Schritt d) trotz des Gehalts an niedrigschmelzendem Fett eine bei Raumtemperatur feste partikelförmige Mischung hergestellt wird. Diese partikelförmige Mischung, auch als Pellets bezeichnet, wird im Intestinaltrakt, insbesondere im Dünndarm, von Säugetieren und Menschen zersetzt und gibt den Inhaltsstoff frei. Es hat sich gezeigt, dass durch Wahl der Art und des Anteils des niedrigschmelzenden Fetts die Freisetzung des Inhaltsstoffs vorbestimmt werden kann.

Der Follistatin enthaltende Rohstoff, hier auch einfach als Follistatin bezeichnet, ist bevorzugt Vollei, Eigelb oder Eiklar aus befruchteten Vogeleiern, insbesondere aus befruchteten Hühnereiern, und/oder rohes tierisches Blutserum, optional behandelt mit dem Schritt des Aufkonzentrierens einer Follistatin enthaltenden Fraktion aus dem Vollei, Eigelb oder Eiklar und/oder tierischem Blutserum durch Fällung einer löslichen Proteinfraktion, die Follistatin enthält, und Abtrennen der gefällten Fraktion vor dem Konservieren des Follistatins.

Der Schritt des Aufkonzentrierens erfolgt bevorzugt mittels Fällung durch Ansäuerung, z.B. auf pH 6 bis pH 5, und/oder Zugabe wasserlöslichen Salzes, z.B. Ammoniumazetat, NaCl, NaHCO₃, KCl und/oder Ammoniumsulfat, und/oder Zugabe eines Lösungsmittels, z.B. Ethanol erfolgt. Die Ansäuerung, insbesondere bei Eiklar, kann durch Fermentation des Ausgangsmaterials erfolgen, während die Konzentration von Zucker durch Zugabe Säure produzierender Bakterien oder Hefen verringert wird. Das Ansäuern kann durch Zugabe von Säure, z.B. Zitronensäure, Milchsäure, Phosphorsäure oder Salzsäure erfolgen. Das Abtrennen einer ausgefällten Proteinfraktion, die Follistatin enthält, kann z.B. aus Eiklar unter natürlicher Schwerkraft erfolgen, bevorzugt durch Zentrifugation bei zumindest 800 x g, bevorzugt zumindest 1000 x g.

Das Konservieren und/oder Sterilisieren des Follistatins, insbesondere des Volleis, Eigelbs oder Eiklars aus befruchteten Vogeleiern und/oder des rohen tierischen Blutserums, optional behandelt mit dem Schritt des Aufkonzentrierens, erfolgt bevorzugt bei einer Temperatur von unterhalb 38 °C, bevorzugt unterhalb 20 °C, bevorzugter unterhalb 10 °C, durch Behandeln mit einem Druck von wenigstens 4000 bar für wenigstens 1 min, vorzugsweise bis 5500-6500 bar, bevorzugter bis 6000 bar für wenigstens 1 min, bevorzugt für 3 min, bevorzugter für wenigstens 5 min, bevorzugt unter Verwendung einer adiabatischen Kompression und Druckentspannung und/oder durch Behandlung mit einem gepulsten elektrischen Feld, insbesondere in einem kontinuierlichen Verfahren, während das Follistatin, insbesondere Eigelb, Eiklar oder Vollei und/oder rohes tierisches Blutserum durch einen Raum gepumpt werden, der von wenigstens zwei Entladungselektroden begrenzt wird, die z.B. eine elektrische Feldstärke von 5 bis 40 kV/cm, z.B. bei 12 kV/cm, bei einer Strömungsgeschwindigkeit von 30 L/h bei einer Temperatur von 30 °C erzeugen, vorzugsweise unter Verwendung von unipolaren Pulsen mit einer Pulsdauer von 5 bis 20 µs bevorzugt von 10 µs, bei einer Wiederholungsrate von 70 bis 200 Hz, insbesondere positiver Rechteckpulse. Bei einem Energiereinsatz von 50 bis 140 kJ/kg ergab sich für Eigelb die Verringerung der bakteriellen Belastung, als KBE bestimmt, um einen Faktor von 10 bzw. 630. Bevorzugt werden die Vogeleier von Hühnern erhalten, d.h. Gallus domesticus.

Diese Schritte zur Konservierung bzw. Sterilisierung von Follistatin, das insbesondere in Eigelb, Eiklar oder Vollei und/oder rohem tierischen Blutserum enthalten ist, sind nichtthermische Verfahrensschritte, d.h. ein Anstieg in der Temperatur, der während der Hochdruckbehandlung und/oder Behandlung im gepulsten elektrischen Feld auftreten kann, ist nicht ursächlich für die Verminderung der Mikroorganismen, insbesondere von Bakterien, um die Konservierung und Sterilisierung zu erreichen. Zusätzlich sind die Ausführungsformen der Konservierungsschritte physikalische Behandlungsverfahren, d.h. ohne Zusatz antimikrobieller chemischer Verbindungen. Entsprechend sind die Ausführungsformen des Konservierungsschritts nicht thermische Verfahrensschritte, die aus physikalischen Behandlungsschritten bestehen, die keine Radikale erzeugen und daher die chemische Struktur der Inhaltsstoffe beibehalten, insbesondere von ungesättigten Fettsäuren und Vitaminen der Zusammensetzung.

Es wurde gefunden, dass die Hochdruckbehandlung und/oder Behandlung im gepulsten elektrischen Feld des Follistatin enthaltenden Materials, das vorzugsweise flüssiges Vollei, flüssiges Eiklar, bevorzugt flüssiges Eigelb und/oder tierisches Blutserum ist, die bakterielle Belastung um einen Faktor von wenigstens 10 wirksam vermindert, vorzugsweise um ein Faktor von wenigstens 100, bevorzugter von wenigstens 1000. Zum Beispiel wurde für die Hochdruckbehandlung eine Verminderung der bakteriellen Belastung auf etwa 50 KBE/g entsprechend einer Verringerung um einen Faktor von 3000 gefunden, wenn von rohem flüssigem Eigelb mit einem natürlichen Bakteriengehalt von 1,5 x 10⁵ KBE/g ausgegangen wurde. Für die Behandlung mit einem gepulsten elektrischen Feld wurde eine Verminderung um einen Faktor von 10 bis zu einem Faktor von 1000 gefunden. Die Verringerung der natürlichen mikrobiologischen Belastung durch die Hochdruckbehandlung und/oder Behandlung im gepulsten elektrischen Feld ist für die Konservierung des Eiklars, Volleis oder Eigelbs ausreichend. Diese Verringerung der mikrobiologischen Belastung wird vorliegend auch als Konservierung und/oder Sterilisierung bezeichnet.

Optional umfasst das Verfahren im Anschluss an den Konservierungsschritt das Trocknen, insbesondere Gefriertrocknen des flüssigen Eipräparats, insbesondere Eigelbs, Eiklares oder Volleis bzw. tierischen Blutserums, was zu einem eihaltigen Pulver führt, insbesondere zu einem Eigelb, Eiklar oder Vollei enthaltendem Pulver oder Tierblutserumpulver, insbesondere zu einem Pulver, das im Wesentlichen aus dem hochdruckbehandelten und/oder mit gepulstem elektrischem Feld behandelten Ei oder Eibestandteilen besteht, z.B. Bestandteilen von Eigelb, Eiklar, Vollei bzw. Blutserum. In Alternative zum Gefriertrocknen kann das Trocknen Wirbelschichttrocknen sein, bevorzugt bei einer Temperatur bei oder unterhalb 42 °C, bevorzugt bei oder unterhalb 40 °C, bevorzugter bei oder unterhalb 38 °C oder bei oder unterhalb 35 °C.

Erfindungsgemäß bevorzugt wird das Follistatin enthaltende Material in flüssiger Form, d.h. ohne Trocknung, im Extruder mit Fett gemischt und diese Mischung wird extrudiert. Denn es hat sich gezeigt, dass auch flüssiges Eigelb, Eiklar, Vollei, bzw. tierisches Blutserum, optional nach einem Schritt des Aufkonzentrierens, nach dem Konservieren und/oder Sterilisieren durch Hochdruckbehandlung und/oder Behandlung im gepulsten elektrischen Feld mit dem Verfahren zu einer lagerstabilen Mischung mit Fett extrudiert werden kann, die biologisch aktives Follistatin enthält.

Es ist gefunden worden, dass das Verfahren zur Herstellung der follistatinhaltigen Zusammensetzung, das einen Konservierungsschritt umfasst, der eine Hochdruckbehandlung und/oder Behandlung im gepulsten elektrischen Feld umfasst oder daraus besteht, optional mit anschließendem Trocknen, bevorzugt ohne Trocknen des Follistatins, sowohl zu einer wirksamen Verringerung der bakteriellen Belastung führt, wie z.B. als lebende Bakterien bestimmt, als auch zu dem Follistatin, das seine biologische Aktivität beibehält, z.B. zu wenigstens 50%, vorzugsweise zu wenigstens 70%, bevorzugter zu wenigstens 80%, bevorzugter zu wenigstens 85%, zu wenigstens 90%, oder zu wenigstens 95%, in Bezug auf die biologische Follistatinaktivität in dem verwendeten Ausgangsmaterial. Die mit dem Verfahren hergestellte Mischung, die das Fett und das Follistatin enthaltende Material bzw. Follistatin enthält oder daraus besteht, wird auch als mit Fett verkapseltes Follistatin bezeichnet und kann auch als Fettkapsel bezeichnet werden.

Insbesondere im Hinblick auf Konservierungsverfahren, die die Bestrahlung verwenden, ist es ein Vorteil des Verfahrens der Erfindung, dass durch den Konservierungsschritt keine Radikale erzeugt werden und daher das erhaltene konservierte flüssige Eigelb, Eiklar oder Vollei oder tierische Blutserum, das vorzugsweise anschließend nicht getrocknet ist, weniger oder keine Radikale und Reaktionsprodukte von Radikalen enthält. Zum Beispiel enthält das konservierte flüssige Eigelb, Eiklar oder Vollei als auch das getrocknete, vorzugsweise gefriertrocknete konservierte Eigelb, Eiklar oder Vollei die ungesättigten Fettsäuren des Eigelbs im Wesentlichen in ihrem natürlichen Zustand und Zusammensetzung, z.B. ohne Veränderungen ihrer Doppelbindungen. Entsprechend enthält die Zusammensetzung, die durch das Verfahren der Erfindung erhältlich ist, bevorzugt die ungesättigten Fettsäuren des Eigelbs ohne Veränderungen ihrer Doppelbindungen, d.h. in ihrem natürlichen biologischen Zustand.

Vorzugsweise wird in dem Verfahren kein chemischer Konservierungsstoff zugesetzt, z.B. wird kein antimikrobielles Mittel zugesetzt. Wahlweise wird ein Antioxidanz zugesetzt, z.B. Ascorbinsäure oder ein neutrales Salz dieser. Vorzugsweise ist das Vollei, Eiklar, bevorzugter Eigelb bzw. tierische Blutserum nur frei von zugesetzten Inhaltsstoffen, z.B. wird das Vollei, Eiklar oder bevorzugter Eigelb bzw. das tierische Blutserum, optional nach Aufkonzentrieren, nur den physikalischen Verfahrensschritten unterworfen, die das Unterwerfen des flüssigen Volleis, Eiklars oder flüssigen Eigelbs bzw. tierischen Blutserums unter die Hochdruckbehandlung und/oder die Behandlung im gepulsten elektrischen Feld umfassen und bevorzugt daraus bestehen, optional gefolgt von Trocknen, z.B. Gefriertrocknen oder Wirbelschichttrocknen, oder bevorzugt in flüssiger Form.

Für die Hochdruckbehandlung ist es bevorzugt, dass das flüssige Vollei, Eiklar oder flüssige Eigelb bzw. tierische Blutserum in verschlossenen Behältern mit einer elastischen Wand enthalten ist, z.B. in Kunststoffbeuteln, bevorzugter frei von Gas, bevorzugter entgast. Für ein gasfreies Vollei, Eiklar oder flüssiges Eigelb bzw. tierisches Blutserum in einem Behälter können die Gasblasen vor dem Versiegeln des Containers ausgestoßen werden. Zum Entgasen kann ein verringerter Druck vor der Hochdruckbehandlung angelegt werden, bevorzugt auch vor der Behandlung im gepulsten elektrischen Feld.

Die Hochdruckbehandlung wird allgemein unter Verwendung von Wasser als Druckmedium durchgeführt, das in eine geschlossene Kammer gepumpt wird, die das flüssige Vollei, Eiklar oder flüssige Eigelb bzw. tierische Blutserum enthält, bis der Hochdruck erreicht ist, Aufrechterhalten des Hochdrucks und dann Entspannen des Hochdrucks, z.B. durch Öffnen des Hochdruckbehälters.

Es ist gefunden worden, dass nach der Hochdruckbehandlung in geschlossenen Behältern, z.B. in geschlossenen Polyethylenbeuteln, das Präparat des flüssigen Volleis, Eiklars oder flüssigen Eigelbs stabil ist, z.B. für 12 bis 24 h, bevorzugt für 2 bis 5 d, z.B. bei 5 bis 10 °C, ohne eine drastische Zunahme der bakteriellen Belastung und insbesondere ohne signifikanten Verlust der Follistatinaktivität. Für eine Hochdruckbehandlung wird der adiabatischen Temperaturerhöhung aufgrund des Hochdrucks vorzugsweise durch Kühlen des flüssigen Volleis, Eiklars oder flüssigen Eigelbs auf eine Temperatur entgegengewirkt, die wenigstens 5°C, bevorzugter etwa 10 °C unterhalb der maximalen Temperatur liegt, z.B. unterhalb 38 °C vor der Behandlung. Vorzugsweise wird das flüssige Vollei, Eiklar oder flüssige Eigelb vor der Hochdruckbehandlung und/oder vor der Behandlung im gepulsten elektrischen Feld auf eine Temperatur zwischen 0 und 28 °C gekühlt, bevorzugt auf 5 bis 20 °C, bevorzugter auf maximal 10 °C.

Für eine Behandlung im gepulsten elektrischen Feld wurde gefunden, dass eine kurze Temperaturerhöhung, z.B. auf ein Maximum von 45 °C, bevorzugt auf ein Maximum von 42 °C oder auf 40 °C für maximal 10 s, bevorzugt für maximal 5 oder maximal 2 s zu einem geringen Verlust aktiven Follistatins führt. Entsprechend ist für die Behandlung im gepulsten elektrischen Feld die vorgenannte kurze Erhöhung der Temperatur annehmbar, wenn auch weniger bevorzugt.

Aktives Follistatin wurde durch Größentrennung, z.B. durch Größenausschluss HPLC oder durch SDS-Polyacrylamidgelelektrophorese (SDS-PAGE), wahlweise gefolgt durch Westernblotten und immunpezifischer Detektion unter Verwendung eines anti-Follistatin-Antikörpers bestimmt. Eine Verringerung des größenspezifischen Signals, das in frischem Eigelb aus befruchteten Eiern gefunden wurde, wurde als ein Indikator für die Verminderung der Follistatinaktivität verwendet, da eine Inaktivierung von Follistatin zu einer Veränderung, z.B. Verringerung, der Molekülgröße führt.

Vorzugsweise umfasst das Verfahren einen Schritt des Aufkonzentrierens von Vollei, Eiklar oder Eigelb der befruchteten Eier. Zur Aufkonzentrierung wird die Fraktion von Vollei, Eiklar oder von Eigelb verwendet, die den höheren Anteil von Follistatin enthält, wobei die Fraktion z.B. durch Größentrennung oder durch Dichtetrennung erhalten wird. Die bevorzugte Fraktion ist die Fraktion, die die Eigelbmembran enthält, z.B. erhalten durch Trennen von Eigelb oder Vollei, und die Fraktion die Hagelschnüre enthält, z.B. erhalten durch Trennen von Eiklar oder Vollei. Bevorzugt enthält die bevorzugte Fraktion den überwiegenden Anteil der Eigelbmembranen und/oder der Hagelschnüre von Vollei, Eiklar oder Eigelb, das dem Aufkonzentrierungs- oder Trennungsschritt unterworfen wird. Für die Trennung durch Größentrennung kann Sieben benutzt werden, z.B. mit einer Maschengröße von 0,5 mm bis 2 mm, bevorzugt etwa 0,5 bis 1 mm. Bei Verwendung der Größentrennung ist die bevorzugte Fraktion die Fraktion, die die Eigelbmembran und/oder Hagelschnüre enthält, die die partikuläre oder große Fraktion ist. Zur Trennung durch Dichtetrennung, Zentrifugation, z.B. unter Verwendung eines Zentrifugalseparators. Bei Verwendung der Dichtetrennung von Vollei, Eiklar oder Eigelb ist die Fraktion höherer Dichte die bevorzugte Fraktion.

Wahlweise kann das Vollei, Eiklar oder Eigelb vor dem Schritt des Aufkonzentrierens des Volleis, Eiklars oder Eigelbs der befruchteten Eier durch Abtrennen der Fraktion, die die Eigelbmembran und/oder Hagelschnüre enthält, verdünnt werden, um den Trennungsschritt zu erleichtern, z.B. durch Verwenden von Wasser als ein Verdünnungsmittel, wobei das Wasser wahlweise Salz enthält.

In der Alternative oder zusätzlich zu Vollei, Eiklar oder Eigelb aus befruchteten Eiern kann das Verfahren zur Verwendung von Blutserum von geschlachteten Tieren als Ausgangsmaterial durchgeführt werden. Entsprechend kann das Blutserum das Vollei, Eiklar oder Eigelb in dem Verfahren ersetzen und daher bezieht sich die Beschreibung bei Bezugnahme auf Vollei, Eiklar oder Eigelb auch auf Blutserum. In dieser Ausführungsform umfasst das Verfahren zur Herstellung einer Zusammensetzung, die biologisch aktives Follistatin enthält, den Schritt des Bereitstellens von rohem Tierblutserum und das Unterwerfen des rohen Tierblutserums unter den Konservierungsschritt, während die Temperatur bei oder unterhalb 38 °C gehalten wird, wobei der Konservierungsschritt die Hochdruckbehandlung von mindestens 4500 bar für wenigstens 1 min, eine Behandlung im gepulsten elektrischen Feld von wenigstens 5 kV/cm bei einer Strömungsrate von 30 L/h oder eine Kombination der Hochdruckbehandlung und der Behandlung im gepulsten elektrischen Feld ist, um ein konserviertes flüssiges rohes Tierblutserum bereitzustellen. Der Schritt der Konservierung kann aus der Hochdruckbehandlung bestehen, wobei das rohe Tierblutserum in Behälter gefüllt wird, die Behälter verschlossen werden und dem Hochdruck unterworfen werden. Der Schritt der Konservierung kann aus einer Behandlung des rohen Tierblutserums im gepulsten elektrischen Feld durch ein elektrisches Feld von 5 bis 40 kV/cm bestehen. Im Anschluss an den Konservierungsschritt kann das konservierte rohe Tierblutserum wahlweise getrocknet werden, z.B. durch Gefriertrocknen oder durch Wirbelschichttrocknung. Bevorzugt wird das konservierte Tierblutserum in flüssiger Form, insbesondere ohne Trocknungsschritt, mit Fett verkapselt. Vorzugsweise wird das rohe Tierblutserum zwischen dem Schritt der Hochdruckbehandlung und dem Schritt des Gefriertrocknens in den geschlossenen Behältern transportiert, in denen das rohe Tierblutserum der Hochdruckbehandlung unterworfen wurde.

Als ein besonderer Vorteil der Hochdruckbehandlung von flüssigem Eigelb, Vollei oder Eiklar ist gefunden worden, dass die Bioverfügbarkeit und Verdaulichkeit des Proteins, bevorzugt des gesamten Proteins, verbessert ist. Daher ist das Verfahren, das den Schritt der Hochdruckbehandlung von flüssigem Eigelb, Vollei oder Eiklar umfasst, zur Herstellung einer konservierten Zusammensetzung, die biologisch aktives Follistatin enthält, bevorzugt, wobei in dieser Zusammensetzung das Protein eine erhöhte Bioverfügbarkeit, z.B. eine erhöhte Verdaulichkeit aufweist, z.B. in Bezug zu unbehandeltem flüssigen Eigelb, Vollei oder Eiklar.

Das Mischen von Fett, das einen Schmelzpunkt von zumindest 50 °C aufweist, mit dem Follistatin kann optional, z.B. für einen Anteil oder das gesamte Fett, das den Schmelzpunkt von zumindest 50 °C aufweist, und den gesamten Follistatin, vor dem Extrudieren erfolgen, z.B. in einem Mischer, der ein Pulvermischer oder ein Extruder sein kann, oder das Mischen kann durch gleichzeitiges Zuführen des Fetts und Follistatin in den Extruder erfolgen, in dem die Mischung extrudiert wird. Insbesondere in der Ausführungsform, in der das Mischen des Fetts mit Follistatin in einem Extruder erfolgt, kann das Fett eine Temperatur oberhalb seiner Schmelztemperatur aufweisen oder eine Temperatur bei oder unterhalb seiner Schmelztemperatur. Ein Mischer oder Extruder, in dem das Fett mit Follistatin gemischt wird, kann als ein stromaufwärts angeordneter Abschnitt des Extruders ausgebildet sein, in dem die Mischung anschließend extrudiert wird. Entsprechend können im Verfahren die Schritte a) und c), bevorzugt a), b) und c) gleichzeitig erfolgen.

Die Fettdosierung des Fetts in Schritt a), das bevorzugt einen Schmelzpunkt von zumindest 50 °C aufweist, erfolgt bevorzugt am Einlassende des Extruders, optional kann die Zudosierung des Follistatins im Bereich des Einlassendes des Extruders oder stromabwärts davon erfolgen. Generell dreht die zumindest eine Schnecke des Extruders kontinuierlich während des Verfahrens, z.B. bei gleicher oder veränderlicher Drehzahl.

Das Erwärmen der Mischung beim Extrudieren erfolgt bevorzugt durch Temperieren des Extruders in einem ersten Extruderabschnitt, z.B. durch Erwärmen des Extrudermantels und/oder der Schnecke entlang eines ersten Extruderabschnitts, sowie durch das Drehen der zumindest einen Schnecke des Extruders. Optional ist der erste Extruderabschnitt nicht temperiert, so dass das Erwärmen der Mischung überwiegend oder im Wesentlichen ausschließlich durch mechanischen Energieeintrag der Extruderschnecke erfolgt. Das an die Erzeugung einer fließfähigen Mischung anschließende Kühlen erfolgt bevorzugt innerhalb desselben Extruders in einem zweiten Extruderabschnitt und einem optionalen dritten Extruderabschnitt, die z.B. unmittelbar stromabwärts an den ersten Extruderabschnitt angrenzen. Für das Kühlen wird der zweite Extruderabschnitt und der optionale dritte Extruderabschnitt gekühlt, insbesondere der Mantel und/oder die Schnecke entlang des zweiten und/oder dritten Extruderabschnitts. Der zweite Extruderabschnitt und/oder der dritte Extruderabschnitt kann z.B. auf 10 °C bis 0 °C gekühlt werden, z.B. auf eine Temperatur von bis zu 70 K, von bis zu 50 K, von bis zu 40 K, von bis zu 35 K, von bis zu 30 K, von bis zu 25 K oder bis zu 20 K unterhalb der Schmelztemperatur des Fetts, das in Schritt a) dosiert wurde, bevorzugt unterhalb der Schmelztemperatur der fließfähigen Mischung, gekühlt werden.

Bevorzugt erfolgt die Kühlung des zweiten Extruderabschnitts im Gegenstrom zur Förderrichtung des Extruders. Optional kann der zweite und/oder der dritte Extruderabschnitt gekühlt werden, um die Mischung im Anschluss an den Austritt aus der Extruderdüse auf eine Temperatur, die maximal 35 K, maximal 30 K, maximal 25 K oder maximal 20 K unterhalb der Schmelztemperatur der fließfähigen Mischung oder unterhalb der Schmelztemperatur des in Schritt a) dosierten Fetts liegt, zu bringen. Denn es hat sich gezeigt, dass die Mischung auch bei einer Temperatur unterhalb der Schmelztemperatur mittels des Extruders durch die Düse gepresst wird und sofort anschließend fest ist. Das Zerkleinern erfolgt bevorzugt unmittelbar nach Austritt der Mischung aus der Düse.

Generell kann die Schmelztemperatur der fließfähigen Mischung die Schmelztemperatur des in Schritt a) dosierten Fetts sein, insbesondere für die Zwecke der Temperierung im Verfahren. Die Schmelztemperatur von Fett, das eine Mischung sein kann, die auch als Schmelzbereich bezeichnet wird, wird mittels Differentialscanningkalorimetrie (DSC) bestimmt. Bevorzugt ist die Schmelztemperatur der mittels DSC bestimmte Schmelzbereich.

Das Zerkleinern der Mischung erfolgt unmittelbar nach Austritt der Mischung aus dem Extruder, wobei die Temperatur, auf die die Mischung gekühlt wurde, eine Viskosität einstellt, die den Durchtritt durch eine Formungseinheit, z.B. aus einer Lochplatte, bevorzugt in Kombination mit einer Schneideinrichtung, zulässt. Die Schneideinrichtung kann z.B. rotierende Messer aufweisen, z.B. 2 bis 6 Messer, die mit 100 bis 1500 Upm stromabwärts angrenzend an der Extruderdüse, die z.B. eine Lochplatte ist, entlanglaufen.

Da die Temperatur, auf die die Mischung gekühlt wurde, wenn sie aus der Extruderdüse austritt, um 1 bis 50 K, z.B. bis 40 K oder bis 30 K, z.B. um 5 bis 25 K oder 10 bis 20 K, unterhalb der Schmelztemperatur des Fetts oder der Mischung ist, erstarrt die Mischung nach Durchtritt durch die Formungseinheit bereits ohne oder bei geringer weiterer Kühlung. Die Fließfähigkeit der Mischung auch bei einer Temperatur unterhalb ihrer Schmelztemperatur, z.B. um 20 K oder 10 K bis 1 K unterhalb ihrer Schmelztemperatur, wird gegenwärtig darauf zurückgeführt, dass die Mischung im Extruder unter Druck steht und kontinuierlich geschert wird. Entsprechend ist generell bevorzugt, dass die Mischung im Extruder kontinuierlich geschert wird, z.B. durch kontinuierliche Drehung der Schnecke. Die aus dem Extruder ausgetretene Mischung erstarrt sofort und bildet, insbesondere nach der Zerkleinerung, Stückchen, die vorliegend auch als Pellets bezeichnet werden, und die bei einer Temperatur unterhalb der Schmelztemperatur des Fetts schüttfähig bzw. rieselfähig sind und daher leicht dosierbar und in Lebensmittel einmischbar sind. Die Pellets weisen das mit Fett verkapselte Follistatin auf oder bestehen daraus.

Beim optionalen Schritt h), dem Behandeln der Pellets durch Temperieren, kann auf eine Temperatur temperiert werden, die zumindest 2 bis 10 K unterhalb der Schmelztemperatur des Fetts liegt, das einen Schmelzpunkt von zumindest 50 °C oder zumindest 55 °C, z.B. 50 bis 90 °C, bevorzugt von 55 bis 70 °C, bevorzugter zumindest 60 bis 70 °C aufweist.

Das Verfahren hat den Vorteil, ein Produkt in Form von Pellets herzustellen, die eine hohe Beladung an Follistatin enthaltendem Inhaltsstoff aufweisen, stellvertreten durch konserviertes und/oder sterilisiertes pulverförmiges oder flüssiges Eigelb, Eiklar oder Vollei und/oder tierisches Blutserum, einen Gehalt von zumindest 5 Gew.-%, zumindest 10 Gew.-%, zumindest 20 Gew.-%, zumindest 30 Gew.-%, zumindest 40 Gew.-% oder zumindest 50 Gew.-%, z.B. bis zu 60 Gew.-%, Rest Fett.

Beim Mischen zumindest eines Anteils des Fetts, das in Schritt a) dosiert wird, mit dem Inhaltsstoff, bevor diese in den Extruder dosiert werden, ist das Fett flüssig, bevorzugt pulverförmig und der Inhaltsstoff ist pulverförmig oder flüssig.

Generell sind das Fett und die Inhaltsstoffe für die Ernährung von Mensch oder Tier geeignet, insbesondere lebensmittelgeeignet, bevorzugt natürlichen Ursprungs. Das Fett kann aus nur einer Verbindung oder einer Mischung von zumindest zwei Verbindungen bestehen. Bevorzugt ist das Fett pflanzlichen Ursprungs. Das Fett kann freie Fettsäure, optional als Salz, Monofettsäureglycerid, Difettsäureglycerid und/oder Trifettsäureglycerid und/oder Wachs, oder eine Mischung aus zumindest zweien dieser, bevorzugt gesättigt, sein. Die freie Fettsäure, die bevorzugt gesättigt ist, oder deren Salz kann eine Kettenlänge von z.B. 10 bis 24 C-Atomen, z.B. 12 bis 18 C-Atome aufweisen, Wachs kann aus zumindest einer C₆- bis C₁₆ -Fettsäure, die mit zumindest einem C₂- bis C₄-Alkohol verestert ist, bestehen.

Ein bevorzugtes Fett, das in Schritt a) dosiert wird, weist durch Hydrieren gehärtetes Pflanzenfett und/oder tierisches Fett, z.B. Fischöl, auf oder besteht daraus, z.B. gehärtetes Kokosfett, gehärtetes Palmfett, gehärtetes Rapsfett, gehärtetes Fischöl, oder eine Mischung aus zumindest zweien dieser.

Fett, das in Schritt a) dosiert wird, hat bevorzugt einen Schmelzbereich von 60 bis 90 °C, bevorzugter 60 bis 70 °C, und ist durch vollständiges Hydrieren gehärtet, z.B. Rapsöl, Rübenöl, Sonnenblumenöl, Palmöl, Palmkernfett, Palmkernöl, Rizinusöl, Maiskaimöl, Erdnussöl, Sojaöl, Weizenkeimöl, Olivenöl, Fischöl, Milchfett, jeweils vollständig hydriert, oder eine Mischung aus zumindest zweien dieser.

Das niedrigschmelzende Fett ist nicht hydriert und kann einfach oder mehrfach ungesättigt sein, z.B. Kokosfett, Palmkernfett, Palmfett, Milchfett, Pflanzenöl, z.B. Rapsöl, Olivenöl, Leinöl, Sonnenblumenöl, Hanföl, Erdnussöl, Walnussöl, Weizenkeimöl, Palmöl, Palmkernöl, Distelöl, Sesamöl, Baumwollsamenöl, oder eine Mischung von zumindest zweien dieser.

Das Behandeln der Pellets durch Temperieren auf eine Temperatur von zumindest 2 bis 10 K unterhalb der Schmelztemperatur des in Schritt a) dosierten Fetts und zumindest 50 °C in Schritt h) kann direkt nach Schritt f) oder nach Schritten f) und g) erfolgen. Bevorzugt werden die Pellets vor Schritt h) in Verpackungen abgefüllt, z.B. elastische Beutel, z.B. aus Kunststoff. Das Behandeln der Pellets in Schritt h) führt zu einer signifikanten Reduktion des Keimgehalts, insbesondere von Bakterien. Überraschender Weise hat sich gezeigt, dass diese Behandlung zur Verringerung des Keimgehalts führt, während die Form und die Qualität der Pellets im Wesentlichen unverändert bleibt. Auch die Qualität bzw. biologische Aktivität des in den Pellets enthaltenen Inhaltsstoffs, insbesondere die biologische Aktivität von Follistatin, blieb durch diese Behandlung im Wesentlichen unverändert.

Der Extruder kann genau eine Schnecke aufweisen oder kann zwei oder mehr Schnecken aufweisen, z.B. ein Doppelschneckenextruder oder Planetwalzenextruder sein. Optional weist der Extruder einen am Einlassende angeordneten Abschnitt auf, der zum Zerkleinern von Feststoffen eingerichtet ist, z.B. mit einem geringeren Spalt zwischen Schnecke und Stator als in einem anschließenden ersten Abschnitt und/oder als im daran anschließenden zweiten Abschnitt.

Optional weist der Extruder einen als Planetwalzenextruder ausgebildeten Abschnitt auf, z.B. als am Einlassende angeordneten Abschnitt, als ersten Abschnitt und/oder als zweiten Abschnitt. Der Planetwalzenextruder hat den Vorteil einer sehr genauen Temperaturführung bei geringem Energieeintrag, die vorteilhaft für temperatur- und schersensible Inhaltsstoffe sein kann.

Die Erfindung wird nun genauer anhand eines Beispiels mit Bezug auf die Figur beschrieben, schematisch einen für das Verfahren bevorzugt verwendeten Extruder zeigt.

### Beispiel: Herstellung eines mit Fett verkapselten Inhaltsstoffs

Als Inhaltsstoff wurde Eipulver verwendet. Zur Herstellung wurden befruchtete Hühnereier (Gallus domesticus), erhalten von einem zertifizierten Zuchtbetrieb, verwendet, wobei die Eier nicht bebrütet waren. Die Eier wurden aufgebrochen und automatisch in Eigelb und das Eiweiß getrennt. 3000 L Eigelb wurden als das rohe flüssige Eigelb verwendet, die vorzugsweise durch Rühren homogenisiert wurden und bei 5 bis 10 °C gehalten wurden und unter sterilen Bedingungen in Polyethylenbeutel gefüllt und nach Ausstossen eingeschlossener Luftblasen versiegelt wurden. Diese Polyethylenbeutel konnten ein Volumen von jeweils zwischen 1 L und 50 L, bevorzugt von 5 bis 20 L haben. Die Beutel wurden in einer Hochdruckkammer (NC-Hyperbaric, Spanien) angeordnet. Unter Verwendung von Wasser als Druckmedium wurde der Druck innerhalb 10 bis 20 min auf 6000 bar erhöht. Nach einer Haltezeit von 3 bzw. 5 min wurde der Druck durch Öffnen eines Entspannungsventils entspannt.

Die bakterielle Belastung wurde durch Plattieren von Standardverdünnungen auf Komplettmedium und Zählen im Anschluss an die Kultivierung in einem Brutschrank bei 37 °C für 48 h bestimmt. Aliquots des hochdruckbehandelten Eigelbs wurden bei etwa 5 °C für einige wenige Stunden gehalten und anschließend durch Einfrieren des Eigelbs und Anwenden von Vakuum, um Wasser zu entziehen, gefriertrocknet, während die Temperatur des Eigelbs vorzugsweise nicht 10 °C überstieg, bevorzugt 5 °C, bevorzugt mit Halten des Eigelbs in einem gefrorenen Zustand.

Die mikrobiologische Analyse zeigte, dass die Hochdruckbehandlung sowohl für 3 min als auch für 5 min zu einer drastischen Verringerung der bakteriellen Belastung führte und auch der anschließende Schritt des Gefriertrocknens die bakterielle Belastung weiter verringerte.

**Tabelle 1: Bakterielle Belastung, gemessen als KBE/g**

| Probe | Salmonellen in 25 g Probe | Gesamtzellzahl (KBE/g) |
|---|---|---|
| rohes flüssiges Eigelb | negativ | 1,5 x 10⁵ |
| flüssiges Eigelb nach 6000 bar, 3 min | negativ | 50 |
| flüssiges Eigelb nach 6000 bar, 5 min | negativ | 50 |
| gefriergetrocknetes Eigelb nach 6000 bar, 3 min | negativ | 40 |
| gefriergetrocknetes Eigelb nach 6000 bar, 5 min | negativ | <10 |

| | | |
|---|---|---|
| KBE = koloniebildende Einheiten (lebensfähige Mikroorganismen) | | |

Die Follistatinaktivität in dem flüssigen Eigelb, wie sie durch SDS-PAGE bestimmt wurde, zeigte eine Verringerung um etwa 15%, oder einen Gehalt von 85% aktiven Follistatins auf Basis des Gehalts aktiven Follistatins, wie er durch SDS-PAGE im rohen flüssigen Eigelb bestimmt wurde.

In dem gefriergetrockneten Eigelb war der Gehalt an aktivem Follistatin in Bezug zur Gesamtproteinkonzentration derselbe wie in dem flüssigen Eigelb nach der Hochdruckbehandlung. Dies zeigt, dass der Schritte des Gefriertrocknens die Aktivität des Follistatins nicht wesentlich beeinflusst, z.B. verringert das Gefriertrocknen nicht wesentlich die Konzentration von aktivem Follistatin pro Gesamtproteingehalt.

Als Alternative wurde ein Aliquot des rohen flüssigen Eigelbs, das in Beispiel 1 verwendet wurde, bei einer Strömungsrate von 30 L/h bei 30 °C durch ein gepulstes elektrisches Feld mit einer Feldstärke von 12 kV/cm unter Verwendung unipolarer positiver Pulse mit einer Pulsdauer von 10 µs bei einer Wiederholungsrate von 200 Hz behandelt. Bei einem Energieeintrag von 50 bis 140 kJ/kg wurde die lebensfähige bakterielle Belastung um einen Faktor von 10 bzw. 630 KBE vermindert, wie durch Plattieren von Verdünnungen bestimmt wurde.

Unter Verwendung von SDS-PAGE wurde eine Verringerung von aktivem Follistatin um etwa 15%, oder eine restliche Aktivität von Follistatin von 85% auf Basis des rohen Eigelbs gefunden. Eine thermische Denaturierung des flüssigen Eigelbs wurde im SDS-PAGE nicht beobachtet.

Optional wurde das Eigelb zur Aufkonzentrierung vor der Hochdruckbehandlung bzw. vor der Behandlung mit gepulsten elektrischen Feldern durch Zentrifugation bei 3343 x g für 20 min in eine Fraktion höherer Dichte, die als ein Pellet gesammelt wurde, und in eine Überstandsfraktion niedrigerer Dichte getrennt. Die follistatinreiche Fraktion wurde als die Fraktion hoher Dichte gefunden.

In der Alternative wurde Vollei oder Eiweiß durch Zentrifugation bei 3343 x g für 20 min in eine Fraktion hoher Dichte getrennt, die als ein Pellet gesammelt wurde, und eine Überstandsfraktion niedrigerer Dichte. Die Fraktion höherer Dichte wurde wiederum als die follistatinreiche Fraktion gefunden.

Die Analyse des Follistatingehalts ist nachfolgend gezeigt:

| Fraktion | Follistatin [µg/kg] |
|---|---|
| Eiweiß, vor der Zentrifugation | 15 |
| Eiweiß, Pellet | 33 |
| | |
| Vollei, vor der Zentrifugation | 23 |
| Vollei, Pellet | 41 |
| | |
| Eigelb, vor der Zentrifugation | 4 |
| Eigelb, Pellet | 36 |

Diese Ergebnisse zeigen, dass die Trennung von Eigelb, Vollei oder Eiweiß in eine Fraktion höherer Dichte, entsprechend den Eigelbmembranen und Hagelschnüren, zu einer erhöhten Konzentration von Follistatin führt, wobei diese Fraktion nach dem Konservierungsschritt, vorzugsweise mit anschließendem Trocknen, eine Zusammensetzung mit erhöhter Konzentration an Follistatin ergibt.

Vorzugsweise wurde das Eigelb, Vollei oder Eiweiß vor der Trennung nicht homogenisiert, z.B. wurde das Eigelb, Vollei oder Eiweiß durch ein weites Sieb hindurchtreten gelassen oder wurde nur gerührt, um die Eigelbmembranen zu brechen, um das Eigelb austreten zu lassen, vorzugsweise ohne Brechen der Eigelbmembranen oder Hagelschnüre in kleine Stücke.

Das Eigelbpulver wurde mit gehärtetem Pflanzenfett, Schmelztemperatur 65-70°C, zu je 50 Gew.-% bei Raumtemperatur chargenweise zu einer Pulvermischung gemischt wurde. Diese Vormischung wurde in das Einlassende eines Doppelschneckenextruders (Typ 44, Bühler) eindosiert. Das Extrudergehäuse wurde in einem ersten Abschnitt, der an das Einlassende angrenzt, auf 70°C temperiert, in einem anschließenden zweiten Abschnitt auf 30°C und in einem an die Düse angrenzenden dritten Abschnitt auf 5°C temperiert. Dabei strömte das Temperiermedium (1:1 Glykol:Wasser) im ersten und zweiten Abschnitt im Gleichstrom zur Förderrichtung des Extruders, im dritten Abschnitt im Gegenstrom. Die eindosierte Vormischung verflüssigte sich im ersten Abschnitt bei Drehung (ca. 100 Upm) der Schnecken. Die aus der Düsenplatte austretenden Extrudatstränge hatten eine Temperatur von ca. 30 °C wurden mittels rotierender Messer zu Pellets geschnitten. Es wird angenommen, dass die Drehung der Schnecken und der dadurch im Extruder gemessene Druck von 14,7 bar ausreichte, um auch bei einer Temperatur der Mischung unterhalb der Schmelztemperatur des Fettes die Mischung hinreichend plastisch bzw. fließfähig zu halten, dass sie durch die Düse gefördert werden konnte.

Alternativ wurde das Eigelbpulver zu 60 Gew.-% mit 40 Gew.-% des Fetts (Schmelzpunkt 65-70°C) zu einer pulverigen Vormischung gemischt. Der an die Einlassöffnung angrenzende erste Abschnitt des Extruders wurde auf 60°C temperiert, der stromabwärts anschließende zweite Abschnitt auf 25°C, und der anschließende dritte Abschnitt, an den sich die Düsenplatte aus Extruderdüse anschließt, wurde auf 0°C temperiert. Das Temperiermedium strömte im ersten und zweiten Abschnitt im Gleichstrom zur Förderrichtung des Extruders, im dritten Abschnitt im Gegenstrom. Optional war zwischen dem ersten Abschnitt und dem Einlassende noch ein zusätzlicher Extruderabschnitt angeordnet, der nicht temperiert war. Dieser zusätzliche Extruderabschnitt scheint keinen wesentlichen Einfluss zu haben. Unmittelbar nach Austritt aus der Düsenplatte wurde für die Extrudatstränge aus der Mischung eine Temperatur von 24,3°C gemessen, ca. 40 bis 45 K unterhalb der Schmelztemperatur des Fetts. Der Druck im Extruder betrug ca. 16 bis 17 bar.

Weiter alternativ wurde der an das Einlassende angrenzende erste Abschnitt des Extrudergehäuses nicht durch Temperiermittel temperiert, der anschließende zweite Abschnitt wurde auf 70°C temperiert, der anschließende dritte Abschnitt auf 30°C temperiert, ein anschließender vierter Abschnitt, an den sich die Düsenplatte anschloss, wurde auf 5°C temperiert. Dabei strömte das Temperiermedium im Gegenstrom zur Förderrichtung des Extruders. Es wurde wiederum eine pulverförmige Vormischung aus Eigelbpulver und pulverförmigem Pflanzenfett (Schmelztemperatur 65-70 °C), je 50 Gew.-%, hergestellt, die in das Einlassende des Extruders eindosiert wurde. Im Extruder wurde ein Druck von 14,7 bar gemessen, am Extruderauslass hatte die Mischung eine Temperatur von maximal 40 K oder von maximal 30 K, z.B. ca. 35 bis 40 K, unterhalb der Schmelztemperatur des Fetts.

Das jeweilige pelletförmige Produkt aus in dem Fett verkapseltem trockenen Eigelb war gegen Feuchtigkeit stabil und schüttfähig.

### Beispiel 2: Herstellung eines mit Fett verkapselten flüssigen Inhaltsstoffs

Als Beispiel für einen flüssigen Inhaltsstoff wurde nach einem Verfahren von Beispiel 1 konserviertes bzw. sterilisiertes Flüssigeigelb verwendet, als Fett Pflanzenfett (Schmelztemperatur 65-70°C). Der Doppelschneckenextruder (Typ 44, Bühler) wurde in einem an das Einlassende angrenzenden ersten Abschnitt auf 60°C temperiert, in dem stromabwärts angrenzenden zweiten Abschnitt auf 10°C und in dem daran stromabwärts angrenzenden dritten Abschnitt auf 0°C. Im ersten und zweiten Abschnitt strömte das Temperiermedium in derselben Richtung wie die Förderrichtung des Extruders, im dritten Abschnitt strömte das Temperiermedium im Gegenstrom zur Förderrichtung des Extruders. Zwischen dem Einlassende und dem ersten Abschnitt des Extruders war optional ein nicht temperierter Extruderabschnitt angeordnet. Das Fett wurde bei Raumtemperatur als Feststoff, pulverförmig oder stückig, zu 30 kg/h in das Einlassende dosiert. Das flüssige Eigelb wurde kontinuierlich mittels einer Exzenterschneckenpumpe durch einen Anschlußstutzen in den zweiten Abschnitt des Extruders zu 10,5 kg/h dosiert. Die Schnecken wurden zu 100 Upm angetrieben.

Die als Extrudatstrang aus der Düsenplatte austretende Mischung war optisch homogen und hatte eine Temperatur von ca. 19-20°C und damit etwa 45 bis 50 K unterhalb der Schmelztemperatur des Fetts. Im Extruder wurde ein Druck von ca. 11 bar gemessen. Bevorzugt wurden die durch Schneiden des Extrudatstrangs erzeugten Pellets anschließend getrocknet, z.B. bei Raumtemperatur bis 4 °C, bis die Pellets oberflächlich trocken waren.

Dieses Beispiel zeigt, dass das Verfahren zur Herstellung von mit Fett verkapselten flüssigem Eigelb, Eiklar oder Vollei, alternativ tierischem Blutserum geeignet ist.

Bevorzugt wurden die Pellets in luftdichte Kunststoffbeutel als Verpackung gefüllt und durch Temperieren für 3 bis 7 d (Tage) bei 50 bis 60 °C, insbesondere bei 58 °C behandelt. Dabei wies das Fett eine höhere Schmelztemperatur auf, z.B. von ca. 65 - 70 °C.

Für die Extrusion wurde z.B. ein Extruder eingesetzt, der wie in der Figur gezeigt in 4 Zonen unterschiedlich temperiert war. Das Extrudergehäuse was aus 8 Segmenten zusammengesetzt, die Schnecke erstreckte sich über die gesamte Länge des Gehäuses. Am Einlassende (Zone I) wurde das hydrierte Rapsöl (HMF) als Pulver in den Extruder zu 25 bis 40 kg/h, z.B.35 kg/h dosiert, der zweite Abschnitt (Zone II) war auf oberhalb der Schmelztemperatur des hydrierten Rapsöls temperiert, das niedrigschmelzende Fett (LMF) wurde auf 40 bis 45 °C temperiert und flüssig mittels einer Dosierpumpe in den zweiten Abschnitt dosiert (2,3 bis 6,8 kg/h), der dritte Abschnitt (Zone III) und der vierte Abschnitt (Zone IV) waren auf 0 °C temperiert, um die Masse auf eine Temperatur von ca. 17 bis 28 °C, gemessen unmittelbar am Extruderaustritt, zu kühlen und unmittelbar anschließend durch eine Düsenplatte mit Öffnungen mit 2 mm Durchmesser, alternativ 0,5 oder 1 mm Durchmesser, zu pressen und die austretenden festen Stränge mit einem rotierenden Messer zu schneiden. Das Follistatin, pulverförmig oder flüssig, oder alternativ Rote Beete-Pulver (RBP) als Modellsubstanz, wurde mit dem hydrierten Rapsöl in das Einlassende dosiert, oder separat in den ersten Abschnitt.

Die Schnecke wurde zu 100 Upm angetrieben, das rotierende Messer hatte 6 Schneiden, die bei 1200 Upm drehten.

Die aus der Düsenplatte ausgetretene Mischung war fest, konnte in formstabile Pellets geschnitten werden und erforderte keine weitere Kühlung, sondern war bei Raumtemperatur formstabil.

Stellvertretend für Follistatin wurde Rote Beete-Pulver als Inhaltsstoff der Pellets eingesetzt, um die Freisetzung aus Pellets zu prüfen. Vor Beginn der in-vitro-Verdauung wurden 14 g Pellets mit destilliertem Wasser für 2 min gespült, um auf der Oberfläche vorhandenes Rote Beete Pulver abzuspülen und somit die Verkapselungseffizienz in Form der Freisetzung von Rote Beete Pulver aus Pellets mittels photometrischer Bestimmung ermitteln zu können. Der in-vitro Test wurde in Anlehnung an Brodkorb et al, Nature Protocols 2019 durchgeführt. Der in-vitro Test umfasst zunächst eine 3-minütige orale Phase, in der die zuvor gespülten 14 g Pellets mit 14 mL synthetischem Speichel mit pH 7 (SSF) im Reaktor des semi-dynamischen Titrationsgeräts bei 37 °C inkubiert werden. Für die Einleitung der gastrischen Phase werden 17 mL synthetischer Magensaft mit pH 3 sowie Pepsin, gelöst in 3 mL SGF mit pH 4,5, dem oralen Gemisch beigefügt und für 2 h bei 37 °C inkubiert. Nach Ende der gastrischen Phase werden 20 mL des gastrischen Gemisches (die Hälfte der Partikel und die Hälfte der Flüssigkeit) mit 17 mL synthetischer Intestinalflüssigkeit (SDF) mit pH 7 sowie Pancreatin, gelöst in 3 mL SDF, und Gallensalzen (CAS Nr. 8008-63-7, Produkt Nr. SC-214601, erhältlich von Santa Cruz Bioltechnology, Heidelberg) gemischt und für 2 h bei 37 °C inkubiert. Der pH-Wert während der semi-dynamischen Verdauung wird computergesteuert durch die Zugabe von Säure und Base auf pH 7 in der oralen Phase, in der gastrischen Phase für 10 min auf pH 3,2, für 20 min auf pH 2,8, für 40 min auf pH 1,8, für 60 min auf pH 1,7 und für 120 min auf pH 1,5 und während der intestinalen Phase für 120 min auf pH 6,5 eingestellt. Der Wechsel zwischen den einzelnen Verdauungsphasen wird manuell durchgeführt.

Die Freisetzung des Inhaltsstoffs, stellvertreten durch das Rote Beete-Pulver, wurde nach oberflächlichem Waschen von Pellets, um loses Pulver zu entfernen, in in vitro Tests bestimmt. Die Freisetzung wurde photometrisch in der freien Lösung bestimmt.

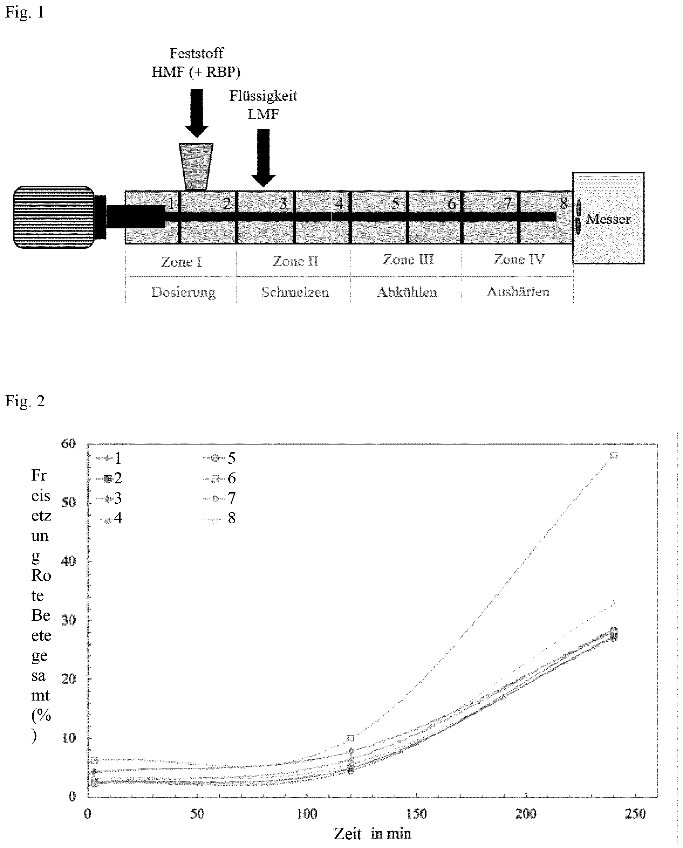

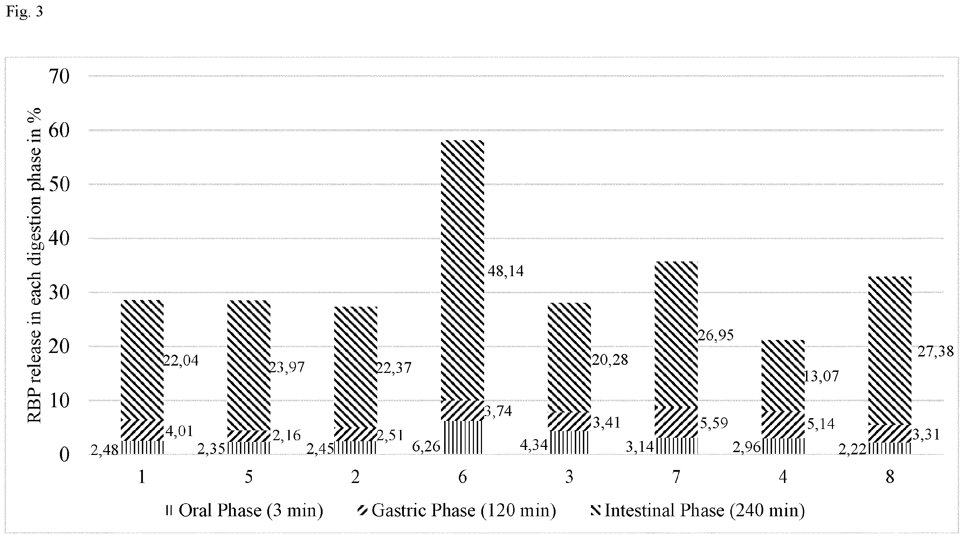

Die Fig. 2 zeigt die Freisetzung des Modellinhaltsstoffs Rote Beete für 12% Rapsöl (1), 12 % Palmkernfett (2), 12 % Kokosfett mit Schmelzpunkt 22-26 °C (3), 12 % Kokosfett mit Schmelzpunkt 23-26 °C (4), 16 % Rapsöl (5), 16 % Palmkernfett (6), 16 % Kokosfett mit Schmelzpunkt 22-26 °C (7), 16 % Kokosfett mit Schmelzpunkt 24-26 °C (8) ist in Fig. 2 über die Zeit des in vitro Verdaus gezeigt, in Fig. 3 ist die Gesamtfreisetzung im Anschluß an die in vitro Verdaus gezeigt.

Dieses Ergebnis zeigt, dass die Pellets eine verzögerte Freisetzung des Großteils den Inhaltsstoffs von bis zu 48% in der intestinalen Phase (Resorptionsort) ergeben, während sie in der oralen und gastrischen Phase eine geringe Freisetzung zeigen bzw. stabil sind und die verkapselte Komponente vor saurem Magensaft schützen.

Die Fettmischung mit 16% niedrigschmelzendem Palmkernfett ist besonders vorteilhaft, da der Anteil an freigesetztem Rote Beete Pulver in intestinaler Phase nahezu doppelt so hoch ist im Vergleich zu Fettkapseln mit Rapsöl oder einem der Kokosfette. Auch ist bei den Pellets mit Palmkernfett eine gute Stabilität und ein guter Schutz während der oralen und gastrischen Phase gefunden worden.

Insgesamt zeigen die Ergebnisse, dass erfindungsgemäß hergestellte Pellets aus mit Fett verkapseltem Inhaltsstoff insbesondere mit einem Anteil an niedrig-schmelzendem Fett eine gezielte Freisetzung des Inhaltsstoffs im Dünndarm erlauben.

## Patentansprüche

1. Verfahren zur Herstellung von mit Fett verkapseltem Follistatin, das die Schritte
a) Dosieren von Fett, das einen Schmelzpunkt von zumindest 40°C aufweist, in einen Extruder,
b) Erwärmen des Fetts, bevorzugt auf zumindest die Schmelztemperatur des Fetts, in dem Extruder bei Drehung der zumindest einen Schnecke,
c) Zudosieren des Follistatins in das Extrudergehäuse zur Herstellung einer fließfähigen Mischung,
d) Kühlen der fließfähigen Mischung in einem stromabwärts angrenzenden Abschnitt des Extrudergehäuses auf eine Temperatur, die in einem Bereich von zumindest 10 K unterhalb der Schmelztemperatur der Mischung liegt,
e) anschließendes Austreten der Mischung durch eine Extruderdüse,
f) Zerkleinern der Mischung nach Austritt aus der Extruderdüse zur Herstellung von Pellets,
g) optional anschließendes Trocknen der Pellets, z.B. bis die Oberfläche trocken ist, bevorzugt bei einer Temperatur unterhalb der Schmelztemperatur des Fetts, aufweist.

2. Verfahren nach Anspruch 1, **dadurch gekennzeichnet, dass** das Follistatin von Vogeleiern stammendes rohes flüssiges Eigelb, Vollei oder Eiklar und/oder rohes tierisches Blutserum ist, von dem wenigstens eine Fraktion, während die Temperatur bei oder unterhalb 38 °C gehalten wird, zur Konservierung und/oder Sterilisation einer Hochdruckbehandlung von wenigstens 4500 bar für wenigstens 1 min, und/oder einer Behandlung mit gepulsten elektrischen Feldern von wenigstens 5 kV/cm bei einer Fließrate von 30 L/h unterworfen wurde.

3. Verfahren nach einem der voranstehenden Ansprüche, **gekennzeichnet durch** das Konzentrieren einer Follistatin enthaltenden Fraktion aus dem löslichen Protein des von Vogeleiern stammenden rohen flüssigen Eigelbs, Volleis oder Eiklars und/oder rohen tierischen Blutserums durch Fällung einer löslichen Proteinfraktion, die Follistatin enthält, und Abtrennen der gefällten Fraktion und **dadurch, dass** im Anschluß an die Abtrennung oder vor der Abtrennung der Follistatin enthaltenden Fraktion diese Fraktion der Konservierung und/oder Sterilisation unterworfen wird.

4. Verfahren nach einem der voranstehenden Ansprüche, **dadurch gekennzeichnet, dass** vor dem Dosieren des Fetts in den Extruder das Follistatin mit dem Fett gemischt wird und eine Mischung, die das Fett und Follistatin enthält, in den Extruder dosiert wird.

5. Verfahren nach einem der voranstehenden Ansprüche, **dadurch gekennzeichnet, dass** nach dem Erwärmen des Fetts, das einen Schmelzpunkt von zumindest 50 °C aufweist, auf zumindest seine Schmelztemperatur in Schritt b) und vor dem Kühlen von Schritt d) zusätzlich ein niedrigschmelzendes Fett, das einen Schmelzpunkt von maximal 40 °C aufweist, zu maximal 20 Gew.-% der Fette in den Extruder dosiert wird.

6. Verfahren nach einem der voranstehenden Ansprüche, **dadurch gekennzeichnet, dass** in Schritt d) das Kühlen erfolgt, bis die Mischung nach Austritt aus der Extruderdüse eine Temperatur aufweist, die im Bereich von 50 K bis 10 K unterhalb der Schmelztemperatur der Mischung liegt.

7. Verfahren nach einem der voranstehenden Ansprüche, **dadurch gekennzeichnet, dass** das die Mischung des mit Fett verkapselten Follistatins zumindest 5 Gew.-% Follistatin enthält.

8. Verfahren nach einem der voranstehenden Ansprüche, **dadurch gekennzeichnet, dass** das Fett hydriertes Pflanzenfett und/oder gehärtetes Fischöl oder eine Mischung aus zumindest zweien dieser ist, und dass das Fett eine Schmelztemperatur von zumindest 40 °C aufweist.

9. Verfahren nach einem der voranstehenden Ansprüche, **dadurch gekennzeichnet, dass** das Follistatin vor dem Zudosieren in den Extruder und vor dem Mischen mit Fett flüssig ist.

10. Verfahren nach einem der voranstehenden Ansprüche, **dadurch gekennzeichnet, dass** das Follistatin vor dem Zudosieren in den Extruder und/oder vor dem Mischen mit Fett getrocknet wird.

11. Verfahren nach einem der voranstehenden Ansprüche, **dadurch gekennzeichnet, dass** das Fett aus zumindest einem hydrierten Pflanzenfett besteht.

12. Verfahren nach einem der voranstehenden Ansprüche, **dadurch gekennzeichnet, dass** die Pellets anschließend an Schritt f) oder anschließend an Schritt g) durch Schritt h) Temperieren auf eine Temperatur von zumindest 2 bis 10 K unterhalb der Schmelztemperatur des Fetts und zumindest 50 °C für zumindest 1 d behandelt werden.

13. Verfahren nach Anspruch 12, **dadurch gekennzeichnet, dass** die Pellets vor dem Temperieren in Schritt h) in luftdichte Verpackungen abgefüllt werden.

14. Verfahren nach einem der voranstehenden Ansprüche, **dadurch gekennzeichnet, dass** die Schmelztemperatur der mittels DSC bestimmte Schmelzbereich ist.

15. Follistatin, das mit Fett verkapselt ist, insbesondere erhältlich nach einem Verfahren gemäß einem der voranstehenden Ansprüche, **dadurch gekennzeichnet, dass** die Mischung des mit Fett verkapselten Follistatins zumindest 5 Gew.-% Follistatin enthält, das konserviertes und/oder sterilisiertes, aus Vogeleiern stammendes rohes flüssiges Eigelb, Vollei oder Eiklar und/oder tierisches Blutserum ist.

16. Follistatin, das mit Fett verkapselt ist, nach Anspruch 15, insbesondere zur Verwendung als Inhaltsstoff zu Lebensmitteln oder Futtermitteln zur Freisetzung des Inhaltsstoffs erst im Dünndarm, **dadurch gekennzeichnet, dass** das Fett eine Mischung eines Fetts, das einen Schmelzpunkt von zumindest 50°C aufweist, mit einem niedrigschmelzenden Fett ist, das einen Schmelzpunkt von maximal 40 °C aufweist und zu 10 bis 20 Gew.-% der Summe der Fette enthalten ist.
